Europäisches Patentamt

European Patent Office

Office européen des brevets

(19)

(11) Publication number: **0 078 162**
**A1**

# (12) EUROPEAN PATENT APPLICATION

(21) Application number: **82305617.1**

(22) Date of filing: **21.10.82**

(51) Int. Cl.³: **C 07 C 69/708**
**C 07 C 67/37**

(30) Priority: **23.10.81 JP 168581/81**

(43) Date of publication of application:
**04.05.83 Bulletin 83/18**

(84) Designated Contracting States:
**BE DE FR GB**

(71) Applicant: **TOA NENRYO KOGYO K.K.**
**1-1 Hitotsubashi, 1-Chome Chiyoda-Ku**
**Tokyo(JP)**

(72) Inventor: **Koyano, Takashi**
**27-4, Honcho 4-chome**
**Nakano-ku Tokyo(JP)**

(72) Inventor: **Tachikawa, Mamoru**
**1902-5, Ooaza Kamekubo Ooi-machi**
**Iruma-gun Saitama-ken(JP)**

(72) Inventor: **Kumagai, Keiji**
**1902-5, Ooaza Kamekubo Ooi-machi**
**Iruma-gun Saitama-ken(JP)**

(74) Representative: **Northover, Robert Frank et al,**
**ESSO CHEMICAL LIMITED ESSO RESEARCH CENTRE**
**P.O. Box 1**
**Abingdon Oxfordshire, OX13 6BB(GB)**

(54) **Process for producing methyl methoxyacetate.**

(57) Methyl methoxyacetate may be produced by reacting formaldehyde or a derivative thereof and carbon monoxide at low pressures and temperature by carrying out the reaction in the presence of a copper (I) carbonyl or a silver carbonyl in a boron trifluoride-methanol solution, and thereafter adding methanol as necessary to complete the reaction.

EP 0 078 162 A1

Process for Producing Methyl Methoxyacetate

The present invention relates to a process for producing methyl methoxyacetate, and, more particularly, to a process for producing methyl methoxyacetate selectively from formaldehyde, carbon monoxide, and methanol.

Methyl methoxyacetate is converted into ethylene glycol when hydrogenated and hydrolyzed. Therefore, it is a precursor for the production of ethylene glycol, and it is also useful as an organic solvent.

According to the prevailing industrial process, the production of ethylene glycol from formaldehyde is accomplished by the steps of reacting formaldehyde and carbon monoxide in the presence of sulfuric acid catalyst to form glycolic acid, esterifying the glycolic acid with methanol to form methyl glycolate, and hydrogenating the methyl glycolate to form ethylene glycol. In this process, the production of the intermediate glycolic acid is carried out at a high temperature under a high pressure, e.g., 200°C and 700 atmospheres. The reaction under such severe conditions in the presence of a corrosive acid requires an expensive apparatus made of corrosion-resistant materials, and consumes a great deal of energy for the generation of high temperature and high pressure. In addition, the production of ethylene glycol from formaldehyde involves many complex reaction steps.

Heretofore, several attempts have been made to solve the above-mentioned problems involved in producing the intermediate for ethylene glycol from formaldehyde.

There are disclosed, for instance, a process for producing glycolic acid by reacting formaldehyde and carbon monoxide at a lower temperature and pressure in the presence of hydrogen fluoride catalyst (Japanese Patent Laid-open Nos. 13719/1976 and 103135/1981); a process for producing glycolic acid by reacting formaldehyde and carbon monoxide in the presence of a catalyst based on a Cu(I) or Ag compound in concentrated sulfuric acid (Japanese Patent Laid-open No. 41327/1976); and a process for reacting formaldehyde and carbon monoxide in sulfuric acid or $BF_3-H_2O$ in the presence of a copper (I) or silver carbonyl compound and then adding methanol, thereby producing an ester of glycolic acid ["Shokubai", 23 (1), 48, (1981)]. Unlike conventional processes, these reactions permit the production of glycolic acid from formaldehyde and carbon monoxide, without requiring high temperature and high pressure. However, they have some disadvantages. That is, in the case where concentrated sulfuric acid is used, the viscosity is so high that sufficient stirring is difficult and the solubility of carbon monoxide in the reaction system limits the reaction rate. In the case where $BF_3-H_2O$ is used, it is comparatively unstable and tends to release $BF_3$ gas. In the case where a hydrogen fluoride catalyst is used, an extremely expensive apparatus is required due to the high corrosiveness of the catalyst. Moreover, the production of ethylene glycol through these reactions requires a step for esterifying glycolic acid, which constitutes a complex process.

In order to establish an industrially feasible process for producing ethylene glycol from formaldehyde applicants carried out investigations, which led to the finding that it is possible to prepare methyl methoxyacetate, which is useful as a precursor for the production of ethylene glycol, directly at a low temperature under a low pressure through the reaction of formaldehyde and carbon monoxide in the presence of a boron trifluoride-methanol complex catalyst containing a copper (I) compound and/or silver compound.

The gist of the present invention resides in a process for producing methyl methoxyacetate which comprises reacting formaldehyde or a derivative thereof and carbon monoxide in the presence of a solution comprising boron trifluoride and methanol, and a copper (I) compound and/or a silver compound. Then a sufficient amount of methanol is added to complete the formation of said product.

### Formaldehyde

The formaldehyde and derivatives thereof used in this invention include methanol solutions of formaldehyde and cyclic or linear polymers, e.g., trioxane and paraformaldehyde, and acetal, e.g., methylal.

### Catalyst

The acid catalyst used in this invention is a boron trifluoride-methanol complex represented by the formula $BF_3 \cdot xCH_3OH$ ($1 \leq x \leq 2$). It can be synthesized by blowing boron trifluoride gas into methanol.

The copper (I) compound and/or silver compound used as catalyst in this invention may be any compound capable of forming copper (I) carbonyl and/or silver carbonyl (described below) in the reaction system. A preferred example of such copper (I) compound is copper oxide and a preferred example of such silver compound is silver oxide or silver borofluoride. The copper (I) compound can be replaced by a 50:50 mixture of metallic copper and copper (II) compound, e.g., a mixture of copper powder and cupric oxide.

The copper (I) carbonyl and/or silver carbonyl is used in the molar ratio of 0.001 to 0.1 for 1 mol of $BF_3 \cdot xCH_3OH$. Thus, relative to the former the latter is in excess.

### Reaction

The process of the present invention is achieved by bringing formaldehyde into contact with the above-mentioned catalyst in the presence of CO.

The reaction temperature is preferably in the range of -10 to +100°C. In this temperature range, the copper (I) compound and silver compound used as co-catalyst are present in the form of copper (I) carbonyl, $Cu(CO)_n^+$

(n = 1 to 4) and silver carbonyl, $Ag(CO)_m^+$ (m = 1 to 2), respectively. Such cation most probably exists as an ion pair with an anionic species of the $BF_3$-methanol complex. The values n and m are close to 1 when the reaction temperature is high, and are close to 4 and 2, respectively, when the reaction temperature is low. Thus, although reaction rate increases at high temperatures, the reaction proceeds advantageously at low temperatures at which the values n and m are high.

The reaction pressure is not specifically limited, but is usually 0.1 to 200 atmospheres, preferably 1 atmosphere to 200 atmospheres, to achieve a balance between the reaction rate and production cost. In the case where a high reaction temperature is employed or a comparatively weak acid catalyst is employed, a high pressure is preferred because a carbonyl co-catalyst does not form readily under such conditions. Usually, the reaction proceeds satisfactorily under low pressures around atmospheric pressure.

According to this invention, copper (I) carbonyl and/or silver carbonyl in $BF_3$-methanol solution (which may be prepared separately in advance or prepared in the reactor) is placed in the carbon monoxide atmosphere. Then, a predetermined quantity of formaldehyde is added slowly with sufficient stirring at a prescribed temperature. The reaction proceeds rapidly and reaches a stage in which the absorption of CO into the reaction solution almost ceases.

After completion of the reaction, the reaction solution is diluted with methanol, and the resulting methyl methoxyacetate is separated from the solvent by distillation together with methanol. It is believed that the methanol for forming the ether methoxy group is derived from $BF_3$-methanol complex during the reaction discussed above and that the ester methyl group is formed from methanol added after said reaction. Prior to distillation, a salt like NaF may be added to decompose the $BF_3$-methanol complex. In this case, the separated $NaBF_4$ is

decomposed to BF$_3$ for recycling. The methyl methoxy-acetate is purified by fractional distillation. Where ethylene glycol is desired as the final product, the methyl methoxyacetate is hydrolyzed to methyl glycolate and then hydrogenated to ethylene glycol with a known catalyst.

The reaction can be accomplished batchwise, semi-batchwise, or continuously. Stirring is desirable to improve the efficiency of contacting reactants with catalysts and to promote the dissolution of carbon monoxide into the reaction system.

Effects of the Invention

The process of the present invention has many advantages. It permits the reaction under mild conditions at low temperature and under low pressure. It permits the direct and selective production of methyl methoxyacetate which is a precursor of ethylene glycol. It permits the reduction of production cost because of less corrosion of apparatus. It simplifies the process. It forms substantially no polymer of glycolic acid, which inevitably decreases the yield in the prior art process.

The invention is now described in more detail, though only by way of illustration, with reference to the following examples.

Example 1

In a 200-ml four-necked flask equipped with a stirrer was placed 0.715 g (0.005 mol) of cuprous oxide. The atmosphere in the flask was replaced by carbon monoxide. Then, into the flask was introduced 50 ml of BF$_3$.CH$_3$OH complex using a syringe. As soon as stirring was started, absorption of carbon monoxide started. Three minutes later, the molar ratio of carbon monoxide absorbed to copper (I) was about 3.2. To this solution was added dropwise with vigorous agitation 7.96 g (0.105 mol) of methylal over 9 minutes. Agitation was continued for 3 hours at room temperature (about 27°C), while keeping the partial pressure of carbon monoxide at 1 atmosphere, so that 1.928 liters of carbon monoxide was absorbed. Four hours later, an additional 0.214 liter of carbon monoxide was absorbed. Finally, 40 ml of methanol was added to the reaction liquid, while cooling with ice water.

Gas chromatography and NMR analysis indicated the production of methyl methoxyacetate in an amount of 10.32 g (94.8% yield based on methylal).

Example 2

Copper carbonyl in $BF_3$-methanol solution was prepared in the same manner as in Example 1, and 3.00 g (0.100 mol) of paraformaldehyde in place of methylal was added to the flask over about 3 minutes. As much carbon monoxide as 1.918 liters was absorbed within 25 minutes. Finally, 40 ml of methanol was added to the reaction liquid, while cooling with ice water. The reaction liquid was found by analysis to contain 7.75 g of methyl methoxyacetate (74.5% yield based on paraformaldehyde) and 1.13 g of methyl glycolate (yield 12.5%).

Example 3

The reaction was carried out in the same manner as in Example 1 using 1.159 g (0.005 mol) of silver oxide in place of cuprous oxide. The molar ratio of carbon monoxide absorbed to silver (I) was about 1.3. To the flask was added dropwise 7.68 g (0.101 mol) of methylal. After agitation for 4 hours, 2.107 liters of carbon monoxide was absorbed. In subsequent 5.5 hours, additional 0.234 liter of carbon monoxide was absorbed. Finally, 40 ml of methanol was added to the reaction liquid, while cooling with ice water. The reaction liquid was found by analysis to contain 9.78 g of methyl methoxyacetate (93.1% yield based on methylal).

Example 4

In a 300-ml glass-lined autoclave equipped with a magnetic stirrer were placed 100 ml of $BF_3 \cdot 1.5CH_3OH$, 1.43 g (0.01 mol) of cuprous oxide, and 6.00 g (0.200 mol) of paraformaldehyde. The atmosphere in the autoclave was replaced by carbon monoxide, and the temperature of the autoclave was raised to 60°C. While keeping this temperature, carbon monoxide was introduced until the pressure increased to 75 kg/cm$^2$. While keeping this pressure, the reaction was carried out for 1 hour with stirring at 1000 rpm. Finally, 40 ml of methanol was added to the reaction liquid, while cooling with ice water. The reaction liquid was found by analysis to contain 19.24 g of methyl methoxyacetate (92.5% yield based on paraformaldehyde) and 0.45 g of methyl glycolate (2.5% yield based on paraformaldehyde).

CLAIMS

1. In the production of methyl methoxyacetate, the step of reacting formaldehyde or a derivative thereof and carbon monoxide in the presence of a solution comprising boron trifluoride and methanol, and a copper compound capable of forming copper (I) carbonyl and/or a silver compound capable of forming silver carbonyl.

2. A process for producing methyl methoxyacetate characterized in that formaldehyde or a derivative thereof is reacted with carbon monoxide in the presence of a solution comprising boron trifluoride and methanol, and a copper (I) compound and/or a silver compound; and adding an appropriate amount of methanol to complete the formation of methyl methoxyacetate.

3. A process according to claim 2 in which copper oxide and/or silver oxide or silver borofluoride is introduced into the reaction system.

4. A process for producing methyl methoxyacetate characterized in that formaldehyde or a derivative thereof is reacted with carbon monoxide in the presence of a solution comprising boron-trifluoride and methanol, and a mixture of copper powder and cupric oxide; and adding an appropriate amount of methanol to complete the formation of methyl methoxy-acetate.

5.   A process for producing methyl methoxyacetate characterized in that formaldehyde or a derivative thereof is reacted with carbon monoxide in the presence of copper (I) carbonyl and/or silver carbonyl and $BF_3$-methanol; and adding sufficient methanol to complete the formation of methyl methoxyacetate.

6.   A process according to claim 5 in which the formaldehyde derivative is selected from the group consisting of trioxane, paraformaldehyde and methylal.

7.   A process according to any of the preceding claims, in which the reaction is carried out at a temperature in the range of $-10°C$ to $+100°C$ and a pressure in the range of 0.1 to 200 atmospheres.

8.   A process according to claim 7, in which the pressure is in the range of atmospheric to 200 atmospheres.

9.   A process according to any of the preceding claims, in which a boron trifluoride-methanol complex is used represented by the formula $BF_3 \cdot xCH_3OH$ wherein $1 \leq X \leq 2$.

10.   A process according to claim 9, in which the formula is $BF_3 \cdot 1.5CH_3OH$.

0078162

European Patent
Office

EUROPEAN SEARCH REPORT

Application number

EP 82 30 5617

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| | --- | | C 07 C 69/708 |
| A | US-A-2 273 269 (R.JOHNSON) *Page 1, left-hand column, line 51 - right-hand column, line 10; page 2, left-hand column, lines 45-62 - right-hand column, line 19* | 1 | C 07 C 67/37 |
| | --- | | |
| A | GB-A- 397 852 (E.I.DU PONT) | 1 | |
| | --- | | |
| A | GB-A- 669 952 (LONZA) | 1 | |
| | ----- | | |

TECHNICAL FIELDS SEARCHED (Int. Cl. ³)

C 07 C 69/00
C 07 C 67/00

The present search report has been drawn up for all claims

| Place of search THE HAGUE | Date of completion of the search 24-01-1983 | Examiner KINZINGER J.M. |
|---|---|---|